(19) Europäisches Patentamt
European Patent Office
Office européen des brevets

(11) EP 0 850 647 B1

(12) **FASCICULE DE BREVET EUROPEEN**

(45) Date de publication et mention de la délivrance du brevet: **26.03.2003 Bulletin 2003/13**

(51) Int Cl.[7]: **A61K 31/565**, A61K 31/58, A61K 31/585

(21) Numéro de dépôt: **97403115.5**

(22) Date de dépôt: **22.12.1997**

(54) **Application de composés stéroides substitués en II pour la fabrication de médicaments ayant une activité estrogène dissociée**

Verwendung von 11-substituierten Steroiden zur Herstellung von Medikamenten mit unterschiedlicher östrogenischen Wirkung

Application of 11-substituted steroids for the preparation of medicaments with dissociated estrogenic activity

(84) Etats contractants désignés:
**AT BE CH DE DK ES FI FR GB GR IE IT LI LU NL PT SE**

(30) Priorité: **23.12.1996 FR 9615828**

(43) Date de publication de la demande:
**01.07.1998 Bulletin 1998/27**

(73) Titulaire: **Aventis Pharma S.A.**
**92160 Antony (FR)**

(72) Inventeurs:
- **Bouali, Yamina**
  **94800 Villejuif (FR)**
- **Nique, François**
  **94170 Le Perreux Sur Marne (FR)**
- **Philibert, Daniel**
  **94210 La Varenne Saint Hilaire (FR)**
- **Teutsch, Jean-Georges**
  **93500 Pantin (FR)**
- **van de Velde, Patrick**
  **75019 Paris (FR)**

(56) Documents cités:
**EP-A- 0 097 572** **EP-A- 0 305 242**
**EP-A- 0 308 345** **FR-A- 2 596 395**

- **QIAN: "Synthesis and biological activities of 11-beta-substituted estradiol as potential antiestrogens" STEROIDS, vol. 55, no. 5, mai 1990, pages 238-241, XP002041035**
- **CVRTILA: "Estrogen and progestin receptors determined in the presence of natural and synthetic ligands and their relationship in primary human breast cancer" PERIOD. BIOL., vol. 88, no. 3, 1986, pages 253-260, XP002041036**
- **GOTTARDIS: "Effect of steroidal and nonsteroidal antiestrogens on the growth of a tamoxifen-stimulated human endometrial carcinoma (EnCa101) in athymic mice" CANCER RES., vol. 50, no. 11, 1990, pages 3189-3192, XP002041037**
- **GOTTARDIS: "Inhibition of tamoxifen-stimulated growth of an MCF-7 tumor variant in athymic mice by novel steroidal antiestrogens" CANCER RES., vol. 49, no. 15, 1989, pages 4090-4093, XP002041038**
- **WEISS NOEL S.: 'Health Consequences of short- and long-term postmenopausal hormone therapy' CLINICAL CHEMISTRY vol. 42, no. 8, 1996, pages 1342 - 1344**
- **LYNDA F. VOIGT ET AL: 'Progestagen supplementation of exogenous oestrogens and risk of endometrial cancer' THE LANCET vol. 338, 1991, pages 274 - 277**
- **SATO ET AL: 'Raloxifene, tamoxifene, nafoxidine, or estrogen effects on reproductive and nonreproductive tissues in ovariectomized rats' FASEB vol. 10, 1996, pages 905 - 912**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 850 647 B1

Printed by Jouve, 75001 PARIS (FR)

## Description

La présente invention concerne l'application de composés stéroïdes substitués en position 11 pour la fabrication de médicaments destinés au traitement hormonal substitutif de la ménopause ne présentant que peu ou pas d'activité estrogène au niveau utérin.

L'ostéoporose est une pathologie qui se caractérise par une réduction quantitative et qualitative du tissu osseux, suffisante pour entraîner des fractures vertébrales ou périphériques, ce de façon spontanée ou à l'occasion de traumatismes minimes. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'estrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité estrogène dissociée, à savoir une activité estrogène au niveau osseux, tout en n'ayant pas ou peu d'activité d'hyperplasie endométriale, ni d'activité de prolifération de tumeur mammaire.

Quian et al, Steroids vol 55 n° 5 mai 1990, 238-241 décrit des composés possèdant une chaine diméthylaminoalkoxy directement sur le carbone 11 de l'estradiol avec une affinité pour le récepteur Estrogène (RE) très réduite. De plus, ces composés sont de purs agonistes au niveau utérin (utérotrphiques) sans aucune activité antagoniste dans ce tissu.

Cvrtila (Period. Biol. Vol 88 n° 3 (1986) 253-260) étudie les concentrations des récepteurs estrogènes et progestogènes dans du tissu tumoral mammaire humain et montre que les ligands synthétiques radiomarqués tels que des dérivés d'estradiol substitués en position 11 par un radical méthoxy sont utilisables pour déterminer les dites concentrations.

L'invention a donc pour objet l'application de composés de formule générale (I) :

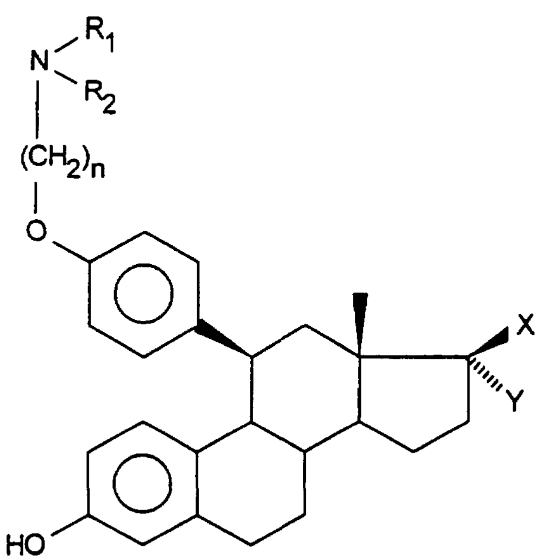

(I)

dans laquelle :

- n est un entier égal à 2 ou 3,
- soit $R_1$ et $R_2$ identiques ou différents représentent un atome d'hydrogène ou un radical alkyle renfermant de 1 à 4 atomes de carbone,
- soit $R_1$ et $R_2$ forment ensemble avec l'atome d'azote auquel ils sont liés un hétérocycle saturé suivant :

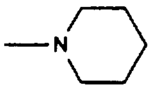 , 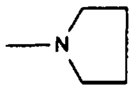 , 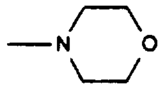 , 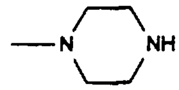 et 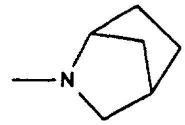

<u>soit</u> X représente un radical hydroxyle, un radical OCOMe ou OCOEt et Y représente un atome d'hydrogène, un radical alkényle ou alkynyle renfermant de 2 à 4 atomes de carbone, et
<u>soit</u> X et Y forment ensemble avec l'atome de carbone qui les portent l'un des cycles suivants :

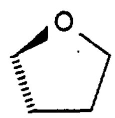 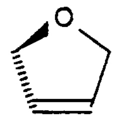 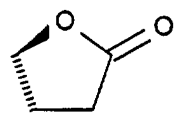

<u>soit</u> X et Y forment ensemble un groupement oxo, ainsi que leurs sels d'addition avec les acides pharmaceutiquement acceptables, pour la préparation d'un médicament destiné au traitement hormonal substitutif de la ménopause, ou de la périménopause.

Par radical alkyle renfermant de 1 à 4 atomes de carbone, on entend les radicaux méthyle, éthyle, propyle, isopropyle, n-butyle, isobutyle, tert-butyle.
Par radical alkényle ou alkynyle renfermant de 2 à 4 atomes de carbone on entend de préférence les radicaux suivants :
$-C{\equiv}CH$, $-C{\equiv}C-Me$, $-C{\equiv}C-CH_2CH_3$, $-CH=CH_2$, $-CH=CH{\sim}Me$, $-CH=H{\sim}CH_2CH_3$.
Le trait ondulé signifie que le radical alkényle présente une configuration (E) ou (Z).
Par sels d'addition avec les acides pharmaceutiquement acceptables, on entend les sels d'addition formés avec des acides minéraux ou organiques sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfonique tels que les acides méthane ou éthane sulfonique, arylsulfoniques, tels que les acides benzène ou paratoluène sulfonique et arylcarboxyliques.
Parmi les symptômes et les conséquences de la ménopause, on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les estrogènes.
L'invention a donc plus particulièrement pour objet l'application de composés de formule (I) tels que définis précédemment pour la préparation d'un médicament destiné à la prévention ou au traitement de l'ostéoporose, ne présentant que peu ou pas d'activité estrogène au niveau utérin.
L'invention a plus particulièrement pour objet l'application telle que définie plus haut des composés de formule (I) dans laquelle n est égal à 2.
L'invention a plus particulièrement pour objet l'application telle que définie plus haut des composés de formule (I) dans laquelle :

n est égal à 2,
<u>soit</u> $R_1$ et $R_2$ identiques ou différents représentent un radical alkyle renfermant de 1 à 4 atomes de carbone,
<u>soit</u> $R_1$ et $R_2$ forment ensemble avec l'atome d'azote auquel ils sont liés un groupement pipéridino, pyrrolidino ou 2-aza-bicyclo(2.2.1)hept-2-yle,
<u>soit</u> X représente un radical hydroxyle et Y représente un atome d'hydrogène, un radical $-C{\equiv}CH$, $-C{\equiv}C-Me$, $-CH=CH{\sim}Me$ (E) ou $-CH=CH{\sim}Me$ (Z)
<u>soit</u> X et Y forment ensemble avec l'atome de carbone qui les portent l'un des cycles tels que définis précédemment, ainsi que de leurs sels d'addition avec les acides pharmaceutiquement acceptables.

L'invention a tout particulièrement pour objet l'application telle que définie plus haut des composés de formule (I) dont les noms suivent :

- 11β-[4-[2-(diméthylamino)éthoxy]phényl]-19-nor-17α-pregna-1,3,5(10)-trièn-20-yne-3,17β-diol,

- 11β-[4-[2-(1-pyrrolidinyl)éthoxy]phényl]-estra-1,3,5(10)-triène-3,17β-diol,
- 11β-[4-[2-(1-pipéridinyl)éthoxy]phényl]-estra-1,3,5(10)-triène-3,17β-diol,
- (17R) 11β-[4-[2-(diméthylamino)éthoxy]phényl]-spiro(estra-1,3,5(10)-triène-17,2'(5'H)-furan)-3-ol,
- (17R) 4',5'-dihydro-11β-[4-[2-(diméthylamino)éthoxy]phényl]-spiro(estra-1,3,5(10)-triène-17,2'(3'H)-furan)-3-ol,
- 11β-[4-[2-(diéthylamino)éthoxy]phényl]-19-nor-17α-pregna-1,3,5(10)-trièn-20-yne-3,17β-diol,
- 11β-[4-[2-(diéthylamino)éthoxy]phényl]-estra-1,3,5(10)-triène-3,17β-diol,
- 11β-[4-[2-(1-pipéridinyl)éthoxy]phényl]-19-nor-17α-prégna-1,3,5(10)-trièn-20-yne-3,17β-diol,
- gamma-lactone de l'acide (17α)-11β-[4-[2-(diéthylamino) éthoxy]phényl]-3,17β-dihydroxy-19-norpregna-1,3,5 (10)-triène-21-carboxylique,
- gamma-lactone de l'acide (17α)-3,17β-dihydroxy-11β-[4-[2-(1-pipéridinyl)éthoxy]phényl]-19-norpregna-1,3,5(10)-triène-21-carboxylique,
- 11β-[4-[2-(diéthylamino)éthoxy]phényl]-3-hydroxy-estra-1,3,5(10)-trièn-17-one,
- 3-hydroxy-11β-[4-[2-(1-pipéridinyl)éthoxy]phényl]-estra-1,3,5(10)-trièn-17-one,
- 11β-[4-[2-[2-aza-bicyclo(2.2.1)hept-2-yl]éthoxy]phényl]-estra-1,3,5(10)-triène-3,17β-diol.

Les composés de formule (I) sont des composés connus. Ils sont spécifiquement décrits ou sont inclus dans des formules générales des brevets suivants :
EP-B-0097572, FR-B-2640977, EP-B-0305242, FR-B-2596395, EP-B-0308345.

Dans ces brevets, il est enseigné que les composés rentrant dans des formules générales très larges peuvent avoir une activité antiglucocorticoïde, antiprogestomimétique, progestomimétique, androgène, antiandrogène, estrogène ou antiestrogène.

La demanderesse a mis en lumière de nouvelles propriétés concernant une sélection très restreinte de ces composés ayant comme point commun, en position 11, un groupement aminoalkyloxy éventuellement substitué et le cycle A aromatique substitué par un groupement OH en position 3.

Il n'est en effet en aucun cas enseigné dans ces brevets que les produits de formule (I) selon l'invention possèdent une activité estrogénique dissociée.

Par activité estrogénique dissociée, on entend une activité estrogénique au niveau osseux tout en ne manifestant qu'une activité minimale au niveau utérin, n'entraînant ainsi pas de prolifération endométriale (activité bien inférieure à celle de l'oestradiol).

Par ailleurs, les composés selon l'invention présentent les avantages suivants :

- Ils présentent une activité antiestrogène au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause chez les femmes à risque de cancer mammaire (antécédents familiaux) qui sont donc exclues d'un traitement substitutif par l'oestradiol,
- Ils entraînent un abaissement du taux de cholestérol sérique à un niveau équivalent à celui induit par l'oestradiol. Ils renforcent ainsi la protection cardio-vasculaire. Enfin, les composés selon l'invention ne présentant pas d'activité estrogène au niveau utérin, ne nécessitent pas d'être administrés en association avec un composé progestomimétique.

L'invention s'étend aux compositions pharmaceutiques renfermant comme principe actif au moins l'un des médicaments tels que définis ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectable, de pommades, de crèmes, de gels, de microsphères, d'implants, d'anneaux intravaginal, de patchs, de sprays, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie utile varie en fonction de l'affection à traiter et de la voie d'administration ; elle peut varier par exemple de 0,5 à 100 mg par jour chez l'adulte par voie orale.

Certains composés de formule (I) sont des composés connus uniquement à titre de composés intermédiaires.

L'invention a donc pour objet à titre de médicaments, les composés de formule (I) dans laquelle $R_1$ et $R_2$ identiques ou différents représentent un radical alkyle renfermant de 1 à 4 atomes de carbone et X et Y forment ensemble un groupement oxo, ainsi que leurs sels d'addition avec les acides pharmaceutiquement acceptables.

L'invention a donc également pour objet les compositions pharmaceutiques les renfermant.

**Tests pharmacologiques**

Molécules étudiées

E2 : Oestradiol

A : 11β-[4-[2-(1-pyrrolidinyl)éthoxy]phényl]-estra-1,3,5(10)-triène-3,17β-diol,
B : 11β-[4-[2-(1-pipéridinyl)éthoxy]phényl]-estra-1,3,5(10)-triène-3,17β-diol,
C : 11β-[4-[2-(diéthylamino)éthoxy]phényl]-estra-1,3,5(10)-triène-3,17β-diol,
D : 11β-[4-[2-(1-pipéridinyl)éthoxy]phényl]-19-nor-17α-pregna-1,3,5(10)-trièn-20-yne-3,17β-diol,
E : 3-hydroxy-11β-[4-[2-(1-pipéridinyl)éthoxy]phényl]-estra-1,3,5(10)-trièn-17-one.

**Effet sur la prolifération de cellules mammaires**

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroïdes (DMEM supplémenté par 5 % de sérum déstéroïdé et sans rouge de phénol). Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à $10^{-10}$M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | E2 | A | B | D |
|---|---|---|---|---|
| Activité | Agoniste | inactif | inactif | inhibiteur |

**Etude de l'impact osseux d'un produit chez la rate femelle ovariectomisée à l'âge de 3 mois.**

Les composés A, B, C, D, E sont testés afin de déterminer leur effet sur la masse osseuse et sur l'activité de formation et de résorption dans le modèle de la rate ovariectomisée à l'âge de 3 mois. Les animaux sont traités en préventif.

| **Animaux :** | |
|---|---|
| Espèce | rat |
| Souche | Sprague-Dawley |
| Sexe | femelle |
| Poids | 250 g à 280 g |
| Nbre d'animaux/groupe | 8 |

**Produits :**

1 - Produit à tester : Produits A, B, C, D, E

- * véhicule(s) : huile de maïs, méthylcellulose 0,5 %
- * dose(s) : une dose par produit testé (0,3 mg/kg/j)
- * nombre d'administrations : une fois/jour ; 5 jours/semaine pendant 4 semaines
- * voie d'administration : voie orale pour les produits
- * volumes : 5 ml/kg (p.o.)

- * délai entre la dernière injection et le sacrifice : 24 heures
- * nombre d'administrations : 20.

2 - Produit de référence : le 17β oestradiol est administré par voie sous cutanée à la dose 0,1 mg/kg/j en solution dans un mélange d'huile de germe de maïs-alcool benzylique (99:1, v/v) sous un volume de 0,2 ml/kg.

**Protocole expérimental**

Animaux

L'étude est réalisée chez des rats femelles ovariectomisées à l'âge de 3 mois. Les animaux sont maintenus dans une pièce climatisée (température 20°C ± 2°C) et groupés par 4 dans des boîtes. Les animaux reçoivent, ad libitum, de l'eau déminéralisée et des aliments comprimés (bouchons : AO4CR-10 UAR).

Chirurgie

Des rats femelles âgées de 3 mois pesant environ 250 g sont ovariectomisées sous anesthésie à l'Imalgène 1000, à la dose de 100 mg/kg par voie intrapéritonéale (i.p.) et sous un volume de 1 ml/kg. Ils reçoivent également du Nembutal (3 mg/kg i.p. sous un volume de 0,3 ml/kg).

Après incision latérale, les plans cutanés et musculaires sont sectionnés. L'exérèse de chaque ovaire se fait après ligature de l'oviducte.

Les rats témoins "SHAM" sont anesthésiés dans les mêmes conditions. Après incision des plans cutanés et musculaires, chaque ovaire est exposé puis replacé in situ.

**Traitement**

Les effets des produits sont déterminés en traitement préventif. Ils sont administrés immédiatement après l'ovariectomie. Les animaux répartis en groupes de 8.

Groupe 1 : rats témoins "SHAM" recevant le ou les véhicules
Groupe 2 : rats témoins "OVX" recevant le ou les véhicules
Groupes X : rats "OVX" recevant respectivement les doses définies du ou des produits à tester.

Prélèvements sanguins

Au terme des 4 semaines (durée de l'étude) les animaux sont décapités par guillotine. Les sérums recueillis après centrifugation sont conservés à -20°C.

Un bilan lipidique sera établi à partir des dosages sériques du cholestérol total, des triglycérides et des phospholipides sur une aliquote de sérum de 500 µl. La baisse du taux de cholestérol sérique est exprimée en % par rapport au taux présenté par les animaux ovariectomisés ne recevant que le solvant.

Prélèvements d'organes

Après sacrifice des animaux, les organes suivants sont prélevés :

- tractus génital

Les utérus sont prélevés. Ces derniers sont pesés. L'augmentation du poids est exprimée, en % du poids de l'utérus des animaux ovariectomisés ne recevant que le solvant.

- au niveau osseux :

La masse osseuse (BMD ou Bone mineral density = densité minérale osseuse) est mesurée par absorptiométrie biphotonique à rayons X en double énergie (DEXA). Les mesures sont réalisées sur les os excisés et débarrassés de tous les tissus mous. La BMD (Bone mineral density) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux et de densité minérale osseuse.

Les résultats sont exprimés en % selon la formule :

$$\frac{\text{BMD produit testé - BMD OVX}}{\text{BMD SHAM - BMD OVX}} \times 100$$

| | Dose | OS TIBIA | UTERUS | Cholestérol |
|---|---|---|---|---|
| | mg/kg | densité % | Poids % | % |
| E2 | 0,1 sc | 105 | 359 | -35 |
| A | 0,3 po | 68 | 70 | -53 |
| B | 0,3 po | 60 | 68 | -50 |
| C | 0,3 po | 63 | 75 | -55 |
| D | 0,3 po | 66 | 88 | -50 |
| E | 0,3 po | 77 | 59 | -52 |
| OVX | | 0 | | |
| SHAM | | 100 | | |
| Nota : pour l'oestradiol : moyenne de 4 essais, pour le composé B, moyenne de 3 essais. | | | | |

Conclusions

Les composés selon l'invention offre une protection osseuse efficace (>60 %), tout en montrant une activité utérotrophique minimale en comparaison de celle provoquée par l'oestradiol. De plus, on observe une baisse significative du taux de cholestérol total.

**Revendications**

**1.** Application de composés de formule générale (I) :

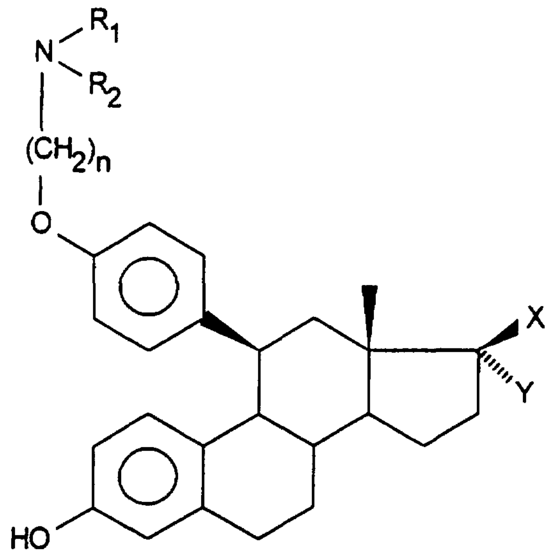

(I)

dans laquelle :

n est un entier égal à 2 ou 3,

soit $R_1$ et $R_2$ identique ou différents représentent un atome d'hydrogène ou un radical alkyle renfermant de 1 à 4 atomes de carbone,
soit $R_1$ et $R_2$ forment ensemble avec l'atome d'azote auquel ils sont liés un hétérocycle saturé suivant :

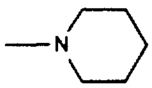 , 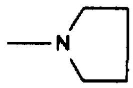 , 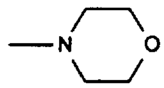 , 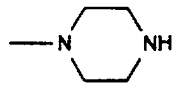 et 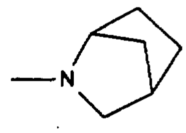

soit X représente un radical hydroxyle éventuellement estérifié et Y représente un atome d'hydrogène, un radical alkényle ou alkynyle renfermant de 2 à 4 atomes de carbone,
soit X et Y forment ensemble avec l'atome de carbone qui les portent l'un des cycles suivants :

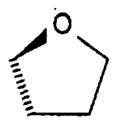 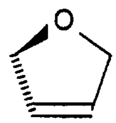 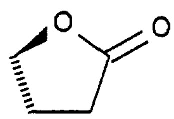

soit X et Y forment ensemble un groupement oxo, ainsi que leurs sels d'addition avec les acides pharmaceutiquement acceptables, pour la préparation d'un médicament destiné au traitement hormonal substitutif de la ménopause, ou de la périménopause.

2. Application selon la revendication 1, **caractérisée en ce que** le médicament est destiné à la prévention ou au traitement de l'ostéoporose.

3. Application selon la revendication 1 ou 2, des composés de formule (I) telle que définie à la revendication 1 dans laquelle n est égal à 2.

4. Application selon la revendication 1 ou 2, des composés de formule (I) telle que définie à la revendication 1 ou 2 dans laquelle :

n est égal à 2,
soit $R_1$ et $R_2$ identique ou différents représentent un radical alkyle renfermant de 1 à 4 atomes de carbone,
soit $R_1$ et $R_2$ forment ensemble avec l'atome d'azote auquel ils sont liés un groupement pipéridino, pyrrolidino ou 2-aza-bicyclo(2.2.1)hept-2-yle,
soit X représente un radical hydroxyle et Y représente un atome d'hydrogène, un radical -C≡CH, -C≡C-Me, -CH=CH~Me (E) ou -CH=CH~Me (Z),
soit X et Y forment ensemble avec l'atome de carbone qui les portent l'un des cycles tels que définis à la revendication 1, ainsi que de leurs sels d'addition avec les acides pharmaceutiquement acceptables.

5. Application selon la revendication 1 ou 2, des composés de formule (I) telle que définie à l'une quelconque des revendications 1 à 3 dont les noms suivent :

- 11β-[4-[2-(diméthylamino)éthoxy]phényl]-19-nor-17α-pregna-1,3,5(10)-trièn-20-yne-3,17β-diol,
- 11β-[4-[2-(1-pyrrolidinyl)éthoxy]phényl]-estra-1,3,5(10)-triène-3,17β-diol,
- 11β-[4-[2-(1-pipéridinyl)éthoxy]phényl]-estra-1,3,5(10)-triène-3,17β-diol,
- (17R) 11β-[4-[2-(diméthylamino)éthoxy]phényl]-spiro(estra-1,3,5(10)-triène-17,2'(5'H)-furan)-3-ol,
- (17R) 4',5'-dihydro-11β-[4- [2- (diméthylamino)éthoxy]phényl]-spiro(estra-1,3,5(10)-triène-17,2'(3'H)-furan)-3-ol,
- 11β-[4-[2-(diéthylamino)éthoxy]phényl]-19-nor-17α-pregna-1,3,5(10)-trièn-20-yne-3,17β-diol,
- 11β-[4-[2-(diéthylamino)éthoxy]phényl]-estra-1,3,5(10)-triène-3,17β-diol,
- 11β- [4- [2- (1-pipéridinyl)éthoxy]phényl]-19-nor-17α-prégna-1,3,5(10)-trièn-20-yne-3,17β-diol,
- gamma-lactone de l'acide (17α)-11β-[4-[2-(diéthylamino) éthoxy]phényl]-3,17β-dihydroxy-19-norpregna-1,3,5 (10)-triène-21-carboxylique,

- gamma-lactone de l'acide (17α)-3,17β-dihydroxy-11β-[4-[2-(1-pipéridinyl)éthoxy]phényl]-19-norpregna-1,3,5(10)-triène-21-carboxylique,
- 11β-[4-[2-(diéthylamino)éthoxy]phényl]-3-hydroxy-estra-1,3,5(10)-trièn-17-one,
- 3-hydroxy-11β-[4- [2-(1-pipéridinyl)éthoxy]phényl]-estra-1,3,5(10)-trièn-17-one,
- 11β-[4-[2-[2-aza-bicyclo(2.2.1)hept-2-yl]éthoxy]phényl]-estra-1,3,5(10)-triène-3,17β-diol.

**6.** Application des composés de formule (I) telle que définie à l'une quelconque des revendications 1 à 5 pour la préparation d'un médicament destiné au traitement des femmes à risque de cancer mammaire.

**7.** A titre de médicaments, les composés de formule (I) telle que définie à la revendication 1 dans laquelle $R_1$ et $R_2$ identiques ou différents représentent un radical alkyle renfermant de 1 à 4 atomes de carbone et X et Y forment ensemble un groupement oxo, ainsi que leurs sels d'addition avec les acides pharmaceutiquement acceptables.

**8.** Compositions pharmaceutiques renfermant comme principe actif au moins l'un des médicaments tels que définis à la revendication 7.

## Claims

**1.** Use of compounds of general formula (I):

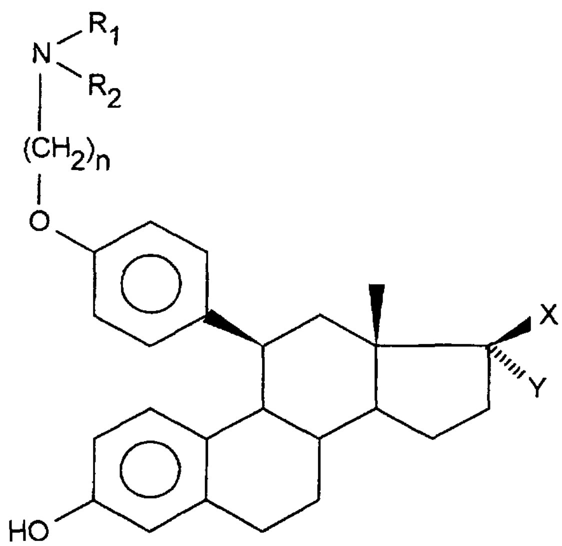

(I)

in which:

n is an integer equal to 2 or 3,
<u>either</u> $R_1$ and $R_2$, identical or different, represent a hydrogen atom or an alkyl radical containing 1 to 4 carbon atoms,
<u>or</u> $R_1$ and $R_2$ form together with the nitrogen atom to which they are linked the following saturated heterocycle:

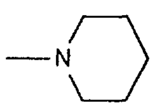 , 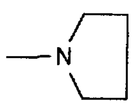 , 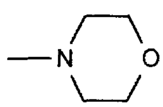 , 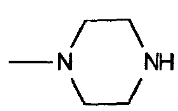 and 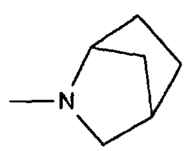

<u>either</u> X represents an optionally esterified hydroxyl radical and Y represents a hydrogen atom, an alkenyl or alkynyl radical containing 2 to 4 carbon atoms,

or X and Y form together with the carbon atom which carries them one of the following rings:

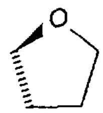 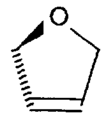 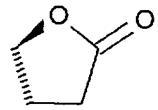

or X and Y together form an oxo group, as well as their addition salts with pharmaceutically acceptable acids, for the preparation of a medicament intended for replacement hormone therapy of the menopause, or the perimenopause.

2. Use according to claim 1, **characterized in that** the medicament is intended for the prevention or the treatment of osteoporosis.

3. Use according to claim 1 or 2, of compounds of formula (I) as defined in claim 1 in which n is equal to 2.

4. Use according to claim 1 or 2, of compounds of formula (I) as defined in claim 1 or 2 in which:

n is equal to 2,
either $R_1$ and $R_2$, identical or different, represent an alkyl radical containing 1 to 4 carbon atoms,
or $R_1$ and $R_2$ form together with the nitrogen atom to which they are linked a piperidino, pyrrolidino or 2-aza-bicyclo(2.2.1)hept-2-yl group,
either X represents a hydroxyl radical and Y represents a hydrogen atom, a -C≡CH, -C≡C-Me, -CH=CH~Me (E) or -CH=CH~Me (Z) radical,
or X and Y form together with the carbon atom which carries them one of the rings as defined in claim 1, as well as their addition salts with pharmaceutically acceptable acids.

5. Use according to claim 1 or 2, of compounds of formula (I) as defined in any one of claims 1 to 3 the names of which follow:

- 11β-[4-[2-(dimethylamino)ethoxy]phenyl]-19-nor-17α-pregna-1,3,5(10)-trien-20-yne-3,17β-diol,
- 11β-[4-[2-(1-pyrrolidinyl)ethoxy]phenyl]-estra-1,3,5(10)-triene-3,17β-diol,
- 11β-[4-[2-(1-piperidinyl)ethoxy]phenyl]-estra-1,3,5(10)-triene-3,17β-diol,
- (17R) 11β-[4-[2-(dimethylamino)ethoxy]phenyl]-spiro(estra-1,3,5(10)-triene-17,2'(5'H)-furan)-3-ol,
- (17R) 4',5'-dihydro-11β-[4-[2-(dimethylamino)ethoxy] phenyl]-spiro(estra-1,3,5(10)-triene-17,2'(3'H)-furan)-3-ol,
- 11β-[4-[2-(diethylamino)ethoxy]phenyl]-19-nor-17α-pregna-1,3,5(10)-trien-20-yne-3,17β-diol,
- 11β-[4-[2-(diethylamino)ethoxy]phenyl]-estra-1,3,5(10)-triene-3,17β-diol,
- 11β-[4-[2-(1-piperidinyl)ethoxy]phenyl]-19-nor-17α-pregna-1,3,5(10)-trien-20-yne-3,17β-diol,
- gamma-lactone of (17α)-11β-[4-[2-(diethylamino) ethoxy]phenyl]-3,17β-dihydroxy-19-norpregna-1,3,5(10)-triene-21-carboxylic acid,
- gamma-lactone of (17α)-3,17β-dihydroxy-11β-[4-[2-(1-piperidinyl)ethoxy]phenyl]-19-norpregna-1,3,5(10)-triene-21-carboxylic acid,
- 11β-[4-[2-(diethylamino)ethoxy]phenyl]-3-hydroxy-estra-1,3,5(10)-trien-17-one,
- 3-hydroxy-11β-[4-[2-(1-piperidinyl)ethoxy]phenyl]-estra-1,3,5(10)-trien-17-one,
- 11β-[4-[2-[2-aza-bicyclo(2.2.1)hept-2-yl]ethoxy]phenyl]-estra-1,3,5(10)-triene-3,17β-diol.

6. Use of the compounds of formula (I) as defined in any one of claims 1 to 5 for the preparation of a medicament intended for the treatment of women at risk from breast cancer.

7. As medicaments, the compounds of formula (I) as defined in claim 1 in which $R_1$ and $R_2$, identical or different, represent an alkyl radical containing 1 to 4 carbon atoms and X and Y together form an oxo group, as well as their addition salts with pharmaceutically acceptable acids.

8. Pharmaceutical compositions containing as active ingredient at least one of the medicaments as defined in claim 7.

## Patentansprüche

**1.** Verwendung von Verbindungen der allgemeinen Formel (I):

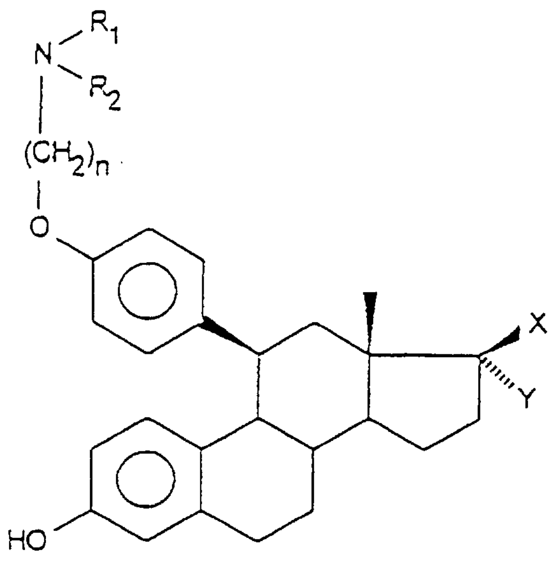

(I)

worin:

n eine ganze Zahl gleich 2 oder 3 ist,
entweder $R_1$ und $R_2$, die gleich oder verschieden sind, ein Wasserstoffatom ode einen Alkylrest mit 1 bis 4 Kohlenstoffatomen darstellen,
oder $R_1$ und $R_2$ zusammen mit dem Stickstoffatom, an das sie gebunden sind, einer gesättigten Heterocyclus bilden entsprechend:

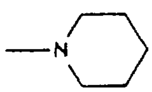 , 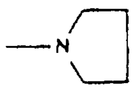 , 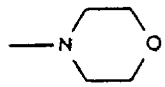 , 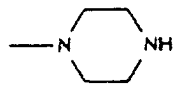 et 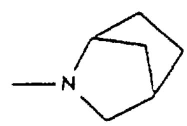

entweder X einen gegebenenfalls veresterten Hydroxylrest darstellt und Y ein Wasserstoffatom, einen Alkenyl- oder Alkinylrest mit 2 bis 4 Kohlenstoffatomen darstellt,
oder X und Y zusammen mit dem Kohlenstoffatom, das sie trägt, einen der folgenden Ringe bilden:

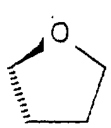 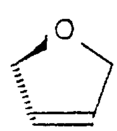 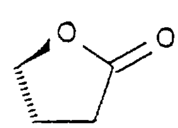

oder X und Y zusammen eine Oxogruppe bilden, sowie ihrer Additionssalze mit den pharmazeutisch annehmbaren Säuren zur Herstellung eines Medikaments, das für die Hormonsubstitutionsbehandlung der Menopause oder der Perimenopause bestimmt ist.

**2.** Verwendung gemäß Anspruch 1, **dadurch gekennzeichnet, dass** das Medikament für die Vorbeugung oder die Behandlung der Osteoporose bestimmt ist.

**3.** Verwendung gemäß Anspruch 1 oder 2 der Verbindungen der Formel (I), wie in Anspruch 1 definiert, worin n gleich 2 ist.

4. Verwendung gemäß Anspruch 1 oder 2 der Verbindungen der Formel (I), wie in Anspruch 1 oder 2 definiert, worin:

n gleich 2 ist,
entweder $R_1$ und $R_2$, die gleich oder verschieden sind, einen Alkylrest mit 1 bis 4 Kohlenstoffatomen darstellen, oder $R_1$ und $R_2$ zusammen mit dem Stickstoffatom, an das sie gebunden sind, eine Piperidino-, Pyrrolidino- oder 2-Aza-bicyclo(2.2.1)hept-2-yl-Gruppe bilden,
entweder X einen Hydroxylrest darstellt und Y ein Wasserstoffatom, einen Rest -C≡CH, -C≡C-Me, -CH=CH~Me (E) oder -CH=CH~Me (Z) darstellt,
oder X und Y zusammen mit dem Kohlenstoffatom, das sie trägt, einen der Ringe, wie in Anspruch 1 definiert, bilden, sowie ihrer Additionssalze mit den pharmazeutisch annehmbaren Säuren.

5. Verwendung gemäß Anspruch 1 oder 2 der Verbindungen der Formel (I), wie in einem der Ansprüche 1 bis 3 definiert, deren Namen folgen:

- 11β-[4-[2-(Dimethylamino)ethoxy]phenyl]-19-nor-17α-pregna-1,3,5(10)-trien-20-in-3,17β-diol,
- 11β-[4-[2-(1-Pyrrolidinyl)ethoxy]phenyl]-estra-1,3,5(10)-trien-3,17β-diol,
- 11β-[4-[2-(1-Piperidinyl))ethoxy]phenyl]-estra-1,3,5(10)-trien-3,17β-diol,
- (17R)-11β-[4-[2-(Dimethylamino))ethoxy]phenyl]-spiro(estra-1,3,5(10)-trien-17,2'-(5'H)-furan)-3-ol,
- (17R)-4',5'-Dihydro-11β-[4-[2-(dimethylamino)ethoxy]phenyl]-spiro(estra-1,3,5(10)-trien-17,2'-(3'H)-furan)-3-ol,
- 11β-[4-[2-(Diethylamino)ethoxy]phenyl]-19-nor-17α-pregna-1,3,5(10)-trien-20-in-3,17β-diol,
- 11β-[4-[2-(Diethylamino)ethoxy]phenyl]-estra-1,3,5(10)-trien-3,17β-diol,
- 11β-[4-[2-(1-Piperidinyl)ethoxy]phenyl]-19-nor-17α-pregna-1,3,5(10)-trien-20-in-3,17β-diol,
- gamma-Lacton der (17α)-11β-[4-[2-(Diethylamino)ethoxy]phenyl]-3,17β-dihydroxy-19-norpregna-1,3,5(10)-trien-21-carbonsäure,
- gamma-Lacton der (17α)-3,17β-Dihydroxy-11β-[4-[2-(1-piperidinyl)ethoxy]phenyl]-19-norpregna-1,3,5(10)-trien-21-carbonsäure,
- 11β-[4-[2-(Diethylamino)ethoxy]phenyl]-3-hydroxy-estra-1,3,5(10)-trien-17-on,
- 3-Hydroxy-11β-[4-[2-(1-piperidinyl)ethoxy]phenyl]-estra-1,3,5(10)-trien-17-on,
- 11β-[4-[2-[2-Aza-bicyclo(2.2.1)hept-2-yl])ethoxy]phenyl]-estra-1,3,5(10)-trien-3,17β-diol.

6. Verwendung der Verbindungen der Formel (I), wie in einem der Ansprüche 1 bis 5 definiert, zur Herstellung eines Medikaments, das für die Behandlung der Frauen mit Brustkrebsrisiko bestimmt ist.

7. Als Medikamente die Verbindungen der Formel (I), wie in Anspruch 1 definiert, worin $R_1$ und $R_2$, die gleich oder verschieden sind, einen Alkylrest mit 1 bis 4 Kohlenstoffatomen darstellen und X und Y zusammen eine Oxogruppe bilden, sowie ihre Additionssalze mit den pharmazeutisch annehmbaren Säuren.

8. Pharmazeutische Zusammensetzungen, die als Wirkstoff wenigstens eins der Medikamente, wie in Anspruch 7 definiert, enthalten.